(19) Europäisches Patentamt
European Patent Office
Office européen des brevets

(11) **EP 3 723 094 A1**

(12) **EUROPEAN PATENT APPLICATION**

(43) Date of publication:
**14.10.2020 Bulletin 2020/42**

(51) Int Cl.:
***G16B 15/20*** (2019.01)

(21) Application number: **20159660.8**

(22) Date of filing: **27.02.2020**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **11.04.2019 JP 2019075588**

(71) Applicant: **FUJITSU LIMITED**
**Kanagawa 211-8588 (JP)**

(72) Inventor: **SATO, Hiroyuki**
**Kawasaki-shi, Kanagawa 211-8588 (JP)**

(74) Representative: **Haseltine Lake Kempner LLP**
**138 Cheapside**
**London EC2V 6BJ (GB)**

(54) **BINDING STRUCTURE SEARCH APPARATUS, BINDING STRUCTURE SEARCH METHOD, AND BINDING STRUCTURE SEARCH PROGRAM**

(57)    A binding structure search apparatus configured to search for a stable binding structure of a molecule, including a creation unit, wherein the molecule is divided at at least one dividing point, and is regarded as a structure having one linear molecular unit including the one dividing point and another linear molecular unit including the one dividing point, the linear molecular unit and the other linear molecular units are arranged at each lattice point of a three-dimensional lattice space that is a set of lattices, the linear molecular units including same dividing points are arranged so as not to overlap with each other, and also arranged in a manner such that the same dividing points are located at the same lattice point, and the creation unit is configured to create a steric structure of the molecule in the three-dimensional lattice space.

FIG. 15

S101 — DIVIDE LINEAR PROTEIN AT DIVIDING POINT

S102 — DEFINE THREE-DIMENSIONAL LATTICE SPACE BASED ON THE NUMBER OF AMINO ACID RESIDUES IN LINEAR MOLECULAR UNIT

S103 — DEFINE SET OF LATTICE POINTS OF DESTINATION OF i-TH AMINO ACID RESIDUE AS $V_i$

S104 — ALLOCATE SPATIAL INFORMATION TO $X_1$ TO $X_n$

S105 — CREATE ISING MODEL

S106 — OBTAIN MINIMUM VALUE OF E(x) BY ANNEALING MACHINE

S107 — OUTPUT RESULT

**Description**

FIELD

The embodiments discussed herein are related to a binding structure search apparatus, a binding structure search method, and a binding structure search program.

BACKGROUND

In recent years, in a scene such as a drug discovery, it may be required to obtain a stable structure of a molecule having a large size by using a calculator (a computer). However, for example, in a case of a large size molecule such as a protein, it may be difficult to search for a stable structure within a practical time in a calculation under careful consideration of all atoms.

Therefore, a technique for reducing the calculation time by roughly capturing the structure of a molecule (coarse graining) has been studied. As a technique for coarse graining of a molecular structure, for example, a technique is known in which each of amino acid residues forming each molecule is treated by performing coarse graining into a point (particle) for a target protein and a peptide molecule bound thereto (for example, see Patent Document 1).

As a technique for coarse graining of the molecular structure, for example, there has been studied a technique in which the molecular structure is subjected to coarse graining into a linear (one series) simple cubic lattice structure based on one dimensional sequence information of an amino acid residue in a protein and treated as a lattice protein. There has been reported a technique for searching for a stable structure at high speed by using the technique of quantum annealing in the lattice protein (see, for example, Non-Patent Document 1).

In a technique for searching for a stable structure in such a lattice protein by an annealing machine, there may be a limitation on the number of arithmetic bits or quantum bits that may be handled due to restrictions of the hardware to be used. The number of bits required to search for a stable structure in the lattice protein increases exponentially with respect to the size (number of amino acid residues) of the protein or peptide.

Therefore, in the above described technique of related art, the number of proteins or peptide amino acid residues as a search target of a stable structure may be limited due to a limitation on the number of bits that may be handled by the hardware to be used. In the above described technique of related art, the number of bits that may simultaneously handle all the amino acids forming proteins or peptides is required, so that the efficiency of calculation may be poor.

The technique of related art for searching for a stable structure in the lattice protein searches for a structure only in consideration of the structure of main chains of proteins. Thus, the structure of side chains of proteins may not be taken into consideration in the technique of related art.

For example, in a case such as a drug discovery, when searching for a stable structure of a protein or peptide which becomes a drug candidate capable of binding to a target protein, it is considered that the structure (position) of the side chain of the amino acid affects the possible structure of the main chain of the amino acid. Therefore, for example, when the technique of the lattice protein is applied to a drug discovery, it may be required to search for a stable structure in a structure including not only the main chain of the amino acid forming the protein but also the side chain of the amino acid.

Patent Document 1: Japanese Laid-open Patent Publication No. 2010-113473.

Non-Patent Document 1: Babbush Ryan, et al., "Construction of Energy Functions for Lattice Heteropolymer Models: A Case Study in Constraint Satisfaction Programming and Adiabatic Quantum Optimization", Advances in Chemical Physics, 155, 201 - 244).

SUMMARY

[TECHNICAL PROBLEM]

In one aspect, it is an object of the present embodiment to provide a binding structure search apparatus, a binding structure search method, and a binding structure search program capable of reducing the number of bits used in searching for a stable binding structure of a molecule by a calculator.

[SOLUTION TO PROBLEM]

According to an aspect of the embodiments, a binding structure search apparatus configured to search for a stable binding structure of a molecule, the binding structure search apparatus includes a memory; and a processor coupled to the memory and configured to: divide the molecule at at least one dividing point, and regard the divided molecule as a structure having one linear molecular unit including the one dividing point and another linear molecular unit including the one dividing point, arrange the linear molecular unit and the other linear molecular units at each lattice point of a three-dimensional lattice space that is a set of lattices, arrange the linear molecular units including same dividing points so as not to overlap with each other, and in a manner such that the same dividing points are located at the same lattice point, and generate a steric structure of the molecule in the three-dimensional lattice space.

[ADVANTAGEOUS EFFECTS OF INVENTION]

In one aspect, it is possible to provide a binding structure search apparatus, a binding structure search method, and a binding structure search program capable of reducing the number of bits used in searching for a stable binding structure of a molecule by a calculator.

BRIEF DESCRIPTION OF DRAWINGS

FIG. 1A is a schematic diagram illustrating an example in which a protein is searched for a stable structure using a coarse graining procedure (Part 1);
FIG. 1B is a schematic diagram illustrating an example in which a protein is searched for a stable structure using a coarse graining procedure (Part 2);
FIG. 1C is a schematic diagram illustrating an example in which a protein is searched for a stable structure using a coarse graining procedure (Part 3);
FIG. 2A is a schematic diagram for explaining an example of a diamond encoding method (Part 1);
FIG. 2B is a schematic diagram for explaining an example of the diamond encoding method (Part 2);
FIG. 2C is a schematic diagram for explaining an example of the diamond encoding method (Part 3);
FIG. 2D is a schematic diagram for explaining an example of the diamond encoding method (Part 4);
FIG. 2E is a schematic diagram for explaining an example of the diamond encoding method (Part 5);
FIG. 3 is a graph illustrating an example of the relationship between the number of amino acid residues and the required number of bits;
FIG. 4 is a schematic diagram for explaining an example of a method for setting a diamond lattice space in the technique of related art;
FIG. 5 is a schematic diagram for explaining an example of a method for setting a diamond lattice space in an example of the technology disclosed herein;
FIG. 6 is a schematic diagram illustrating an example of the arrangement of linear molecular units;
FIG. 7 is a schematic diagram illustrating an example of the structure of a molecule having a plurality of linear molecular units;
FIG. 8 is a schematic diagram illustrating an example in which the linear molecular unit is further divided;
FIG. 9 is a schematic diagram illustrating an example of a coarse grained structure of the same molecule in a case where a structure of a side chain of the amino acid residue is taken into consideration and in a case of not into consideration;
FIG. 10 is a schematic diagram illustrating an example of a case where a stable structure is searched for the molecules illustrated in FIG. 9;
FIG. 11 is a schematic diagram illustrating an example of a state in which a protein subjected to coarse graining in consideration of the structure of the side chain of the amino acid residue is divided into a plurality of linear molecular units;
FIG. 12 is a diagram illustrating a configuration example of a binding structure search apparatus disclosed herein;
FIG. 13 is a diagram illustrating another configuration example of the binding structure search apparatus disclosed herein;
FIG. 14 is a diagram illustrating another configuration example of the binding structure search apparatus disclosed herein;
FIG. 15 is a flowchart illustrating an example of a method for searching for a stable structure of a linear protein;
FIG. 16 is a diagram illustrating an example in which each lattice having a radius r is denoted by $S_r$;
FIG. 17A is a diagram illustrating an example of a set of lattice points of a destination of an amino acid residue (Part 1);
FIG. 17B is a diagram illustrating an example of a set of lattice points of a destination of an amino acid residue (Part 2);
FIG. 17C is a diagram illustrating an example of a set of lattice points of a destination of an amino acid residue (Part 3);
FIG. 17D is a diagram illustrating an example of a set of lattice points of a destination of an amino acid residue (Part 4);
FIG. 18 is a diagram illustrating an example in which $S_1$, $S_2$, and $S_3$ are represented in three dimensions;
FIG. 19A is a diagram illustrating an example of a state in which spatial information is allocated to each of bits $X_1$ to $X_n$ (Part 1);

FIG. 19B is a diagram illustrating an example of a state in which spatial information is allocated to each of the bits $X_1$ to $X_n$ (Part 2);

FIG. 19C is a diagram illustrating an example of a state in which spatial information is allocated to each of the bits $X_1$ to $X_n$ (Part 3);

FIG. 20 is a diagram for explaining an example of $H_{one}$;

FIG. 21 is a diagram for explaining an example of $H_{olap}$;

FIG. 22 is a diagram for explaining an example of $H_{conn1}$ and $H_{conn2}$;

FIG. 23A is a diagram for explaining an example of $H_{pair1}$ and $H_{pair2}$ (Parti);

FIG. 23B is a diagram for explaining an example of $H_{pair1}$ and $H_{pair2}$ (Part2);

FIG. 24 is a diagram illustrating an example of a weight file;

FIG. 25 is an explanatory diagram illustrating an example of conditions for constructing the energy equation (Hamiltonian) of an Ising model;

FIG. 26 is a flowchart illustrating an example of a method for searching for a stable structure of a protein in consideration of the structure of a side chain of an amino acid residue;

FIG. 27 is a diagram illustrating an example of a functional configuration of an optimization apparatus (control unit) used in an annealing method;

FIG. 28 is a block diagram illustrating an example of a circuit level of a transition control unit;

FIG. 29 is a diagram illustrating an example of an operation flow of the transition control unit; and

FIG. 30 is a diagram illustrating an example of the number of lattice points required in searching for stable binding structures of proteins (peptides) and the difference between the searched stable structures in Embodiments 1 and 2, and in the related art.

DESCRIPTION OF EMBODIMENTS

(Binding Structure Search Apparatus)

The binding structure search apparatus disclosed herein is an apparatus for searching for a stable binding structure of molecules. The binding structure search apparatus disclosed herein has a creation unit, preferably includes a calculation unit, and further includes another portion (means) as required.

First, a method for determining a folding structure of a protein by the diamond encoding method, which is one of techniques using the lattice protein, will be described before describing the details of technique disclosed herein.

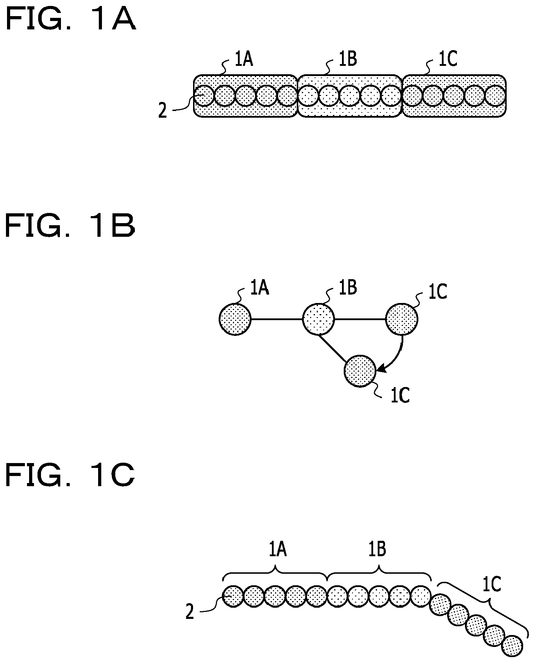

When performing a structural search for the protein (or peptide) using the lattice protein, coarse graining of the protein is firstly performed. As illustrated in FIG. 1A, the coarse graining of the protein is performed by making a coarse-grained model by performing coarse graining on atoms 2 constituting the protein into coarse-grained particles 1A, 1B, and 1C, each of which is a unit for each amino acid residue.

Next, the created coarse-grained model is used to search for a stable binding structure. FIG. 1B illustrates an example of a case where the binding structure in which the coarse-grained particles 1C are located at an end point of an arrow is stable. The stable binding structure is searched by the diamond encoding method described later.

As illustrated in FIG. 1C, the coarse-grained model is returned to the all-atoms model based on the stable binding structure searched by using the diamond encoding method.

For example, the diamond encoding method is a method of fitting a particle (coarse-grained model) subjected to coarse graining on a chain amino acid forming a protein to a lattice point of a diamond lattice, and it is possible to express the three-dimensional structure of a protein.

In the following description, for simplification of explanation, the diamond encoding method applied to a two dimensional case will be described by way of example.

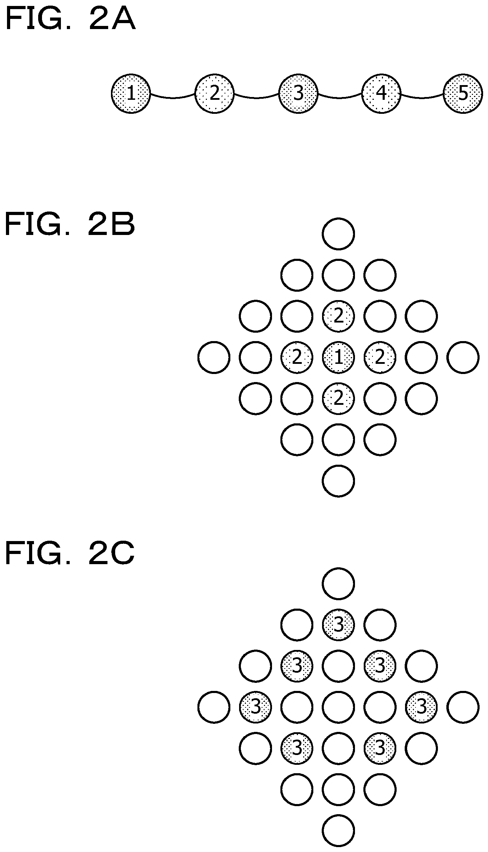

FIG. 2A illustrates an example of a structure in which a linear pentapeptide having five amino acid residues bound to each other has a linear structure. In FIG. 2A to FIG. 2E, numbers in circles represent a number of an amino acid residue in the linear pentapeptide.

In the diamond encoding method, first, when an amino acid residue of a number 1 is arranged at the center of the diamond lattice, as illustrated in FIG. 2A, a place where an amino acid residue of a number 2 may be arranged is limited to a place (place numbered 2) illustrated in FIG. 2B, which is adjacent to the center.

Subsequently, a place where an amino acid residue of a number 3 bound to the amino acid residue of the number 2 may be arranged is, in FIG. 2C, limited to a place (place numbered 3) adjacent to the place numbered 2 in FIG. 2B.

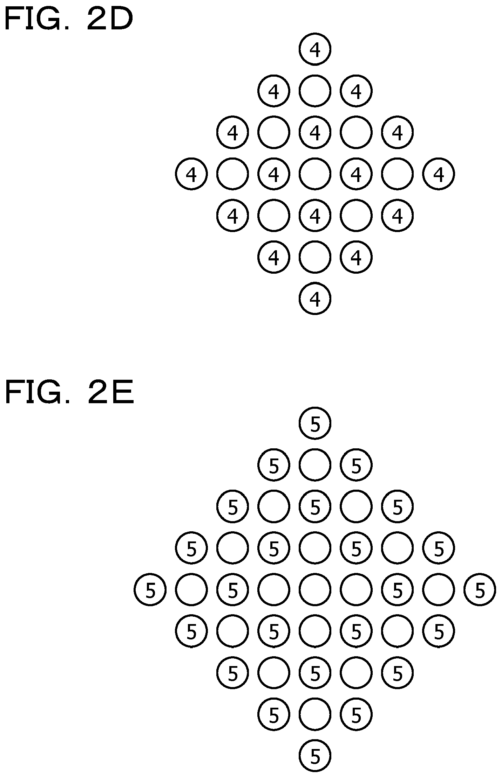

A place where an amino acid residue of a number 4 bound to the amino acid residue of the number 3 may be arranged is, in FIG. 2D, limited to a place (place numbered 4) adjacent to the place numbered 3 in FIG. 2C.

A place where an amino acid residue of a number 5 bound to the amino acid residue of the number 4 may be arranged is, in FIG. 2E, limited to a place (place numbered 5) adjacent to the place numbered 4 in FIG. 2D.

By linking the specified places where the amino acid residues are arranged in the order of the amino acid residue numbers, the structure of the protein subjected to coarse graining may be expressed.

In such a technique of related art as described above, proteins as a search target of a stable binding structures are treated as those in which the amino acid residues are simply bound to each other in a chain state. Therefore, when a stable structure of a protein is searched using an annealing machine or the like, the number of bits that may simultaneously handle all the amino acids forming the protein are required.

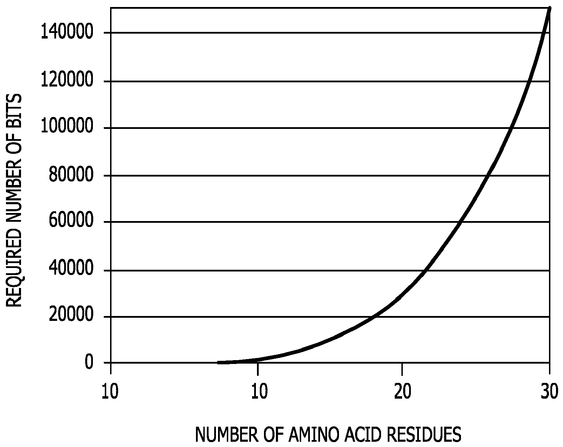

In a case where proteins are treated as those in which the amino acid residues are simply bound in a chain state, the number of bits required for searching the structure increases exponentially as the number of the amino acid residues forming the protein increases as illustrated in FIG. 3, for example.

In a case where a stable structure of a protein is searched using an annealing machine or the like, there may be a limitation on the number of arithmetic bits or quantum bits that may be handled due to restrictions on the hardware to be used. Therefore, there are cases where the number of the amino acid residues of proteins or peptides as a search target of a stable structure is limited due to the limitation of the number of bits that may be handled by the hardware to be used.

In recent years, attention has been focused on so-called medium molecular drug discovery, and it may be required to search for stable structures of proteins or peptides of about 50 residues from several residues, which become a medium molecular drug candidate. In this case, in such a technique of related art as described above, there may be a case where a stable structure of proteins or peptides of about 50 residues from several residues, which become the medium molecular drug candidate may not be searched due to the limitation on the number of bits which may be handled by the hardware to be used.

In the above described technique of related art, the number of bits that may simultaneously handle all the amino acids forming proteins or peptides is required, so that the efficiency of calculation may be poor.

Therefore, the inventors have devised a technique disclosed herein by making extensive studies on an apparatus or the like capable of reducing the number of bits used for searching for a stable binding structure of a molecule by a calculator. For example, the present inventors have found that the molecule is divided at least one dividing point and is regarded as a structure having one linear molecular unit including one dividing point and another linear molecular unit including one dividing point, one linear molecular unit and the other linear molecular unit are arranged at each lattice point of a three-dimensional lattice space, which is a set of lattices and the linear molecular units including the same dividing point are arranged so as not to overlap with each other and also arranged in a manner such that the same dividing points are located at the same lattice point to thereby create a steric structure of the molecule in the three-dimensional lattice space, whereby it is possible to reduce the number of bits used for searching for a stable binding structure of the molecule by a calculator.

Hereinafter, an example of the technique disclosed herein will be described with reference to the drawings.

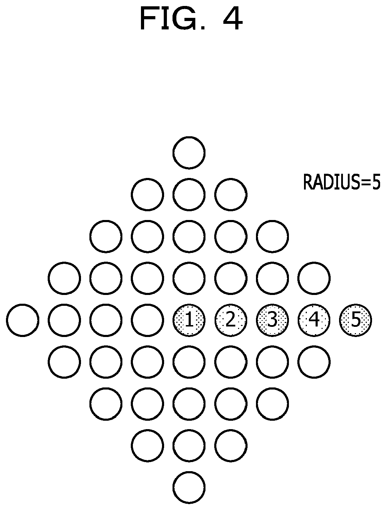

As illustrated in FIG. 4, in the technique of related art, when a stable structure of a linear pentapeptide having five amino acid residues bound thereto is searched, a diamond lattice space having a radius (n) is set according to the number (n) of the amino acid residues to be bound. Therefore, in the example illustrated in FIG. 4, it is required to prepare 41 lattice points, and it is required to prepare an arithmetic bit or a quantum bit in an annealing machine or the like according to the number of lattice points. In the following, the arithmetic bits and the quantum bits are sometimes simply referred to as "bits".

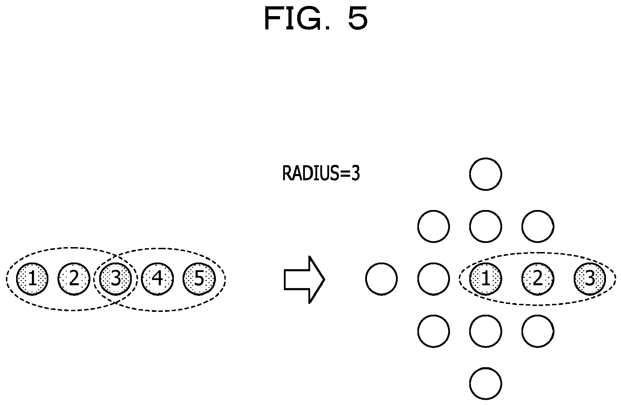

On the other hand, in one example of the technique disclosed herein, as illustrated in FIG. 5, the third amino acid residue of the linear pentapeptide is regarded as a dividing point, and the linear pentapeptide is regarded as a structure having two linear molecular units. For example, a linear pentapeptide as a molecule is divided at one dividing point and is regarded as a structure having one linear molecular unit including one dividing point and another linear molecular unit including one dividing point.

In this way, in one aspect, the technique disclosed herein may reduce the number of lattice points in a diamond lattice space, which is an example of a three-dimensional lattice space, used in searching for a stable binding structure of molecules, and reduce the number of required bits. For example, more specifically, in the example illustrated in FIG. 5, since one linear molecular unit is formed of three amino acid residues, there are 13 lattice points required to search for the structure of one linear molecular unit. Therefore, when a lattice point is prepared for each linear molecular unit, the number of lattice points required to search for the structure of the entire molecule is 26.

In this manner, in the example illustrated in FIG. 5, it is found that the structure may be searched with a smaller number of lattice points than in the example of the related art illustrated in FIG. 4. In this way, in the example of FIG. 5, the number of lattice points required for the search of the structure may be reduced, so that the number of required bits may be reduced. In the technique disclosed herein, in one aspect, since the structure search in the three-dimensional lattice space is actually performed, it is possible to reduce the number of lattice points required in a larger ratio than the examples illustrated in FIGs. 4 and 5, and thus, for example, the number of required bits may be reduced to 1/3 or less.

For example, according to the technique disclosed herein, in one aspect, a molecule is divided at one dividing point, and is regarded as a structure having one linear molecular unit including one dividing point and another linear molecular unit including one dividing point (a structure in which one linear molecular unit and another linear molecular unit are bound to each other), whereby the number of bits required to search for the structure may be reduced.

In one example of the technique disclosed herein, one linear molecular unit and another linear molecular unit are arranged, and the linear molecular units including the same dividing point are arranged so as not to overlap with each other and also arranged in a manner such that the same dividing points are located at the same lattice point.

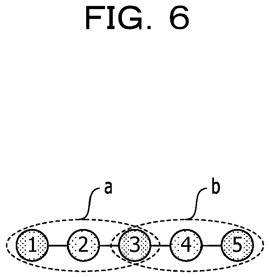

For example, in the example illustrated in FIG. 6, based on information about the amino acid binding order (amino acid sequence) in the protein for searching for the structure, the amino acid of the number 3 serving as the dividing point is arranged so as to be located at the same lattice point in a linear molecular unit a and a linear molecular unit b. In the example illustrated in FIG. 6, for example, the amino acid residues of the numbers 1 and 2 included in the linear molecular unit a are arranged so as not to overlap with the amino acid residues of the numbers 4 and 5 included in the linear molecular unit b.

In this way, in one aspect, the technique disclosed herein may create a structure in which the linear molecular units obtained by dividing a protein for searching for a structure are arranged so as to have a consistent structure as the protein. Accordingly, in the technique disclosed herein, in one aspect, in a case where a ground state search is performed using an annealing machine or the like, while suppressing the number of bits required to search for the structure of the molecule, it is possible to calculate the steric structure of the molecule having a minimum energy with no contradiction as the structure of the molecule.

For example, a specific method for arranging the linear molecular units including the same dividing point so as not to overlap with each other and also arranged in a manner such that the same dividing points are located at the same lattice point will be described later.

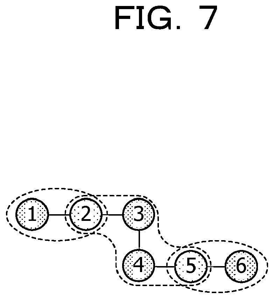

The structure (shape) of the linear molecular unit is not particularly limited, and may not be a straight line. For example, as illustrated in FIG. 7, the linear molecular unit in the molecule for searching for the structure may have a curved structure.

In one example of the technique disclosed herein, a linear molecular unit is regarded as a structure composed of further a plurality of small linear molecular units. For example, in one example of the technique disclosed herein, the linear molecular unit is further divided using particles located at positions other than ends of one linear molecular unit as dividing points.

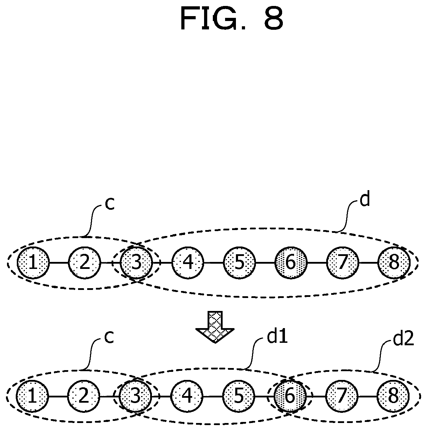

For example, more specifically, as illustrated in the left part of FIG. 8, when the molecule formed by eight particles is divided using a particle of the number 3 as the dividing point, the molecule may be regarded as a structure having a linear molecular unit c formed by three particles and a linear molecular unit d formed by six particles.

As illustrated in the right part of FIG. 8, when the linear molecular unit d formed by the six particles is further divided using the particle of a number 6 as a dividing point, the linear molecular unit d may be regarded as a structure composed of a linear molecular unit d1 formed by four particles and a linear molecular unit d2 formed by three particles. In this case, the molecule illustrated in the right part of FIG. 8 may be regarded as a structure composed of the linear molecular unit c, the linear molecular unit d1, and the linear molecular unit d2.

Since the number of lattice points required for searching for a stable structure is determined according to the number of particles of a linear molecular unit having the largest number of particles, the number of lattice points required for searching for a structure may be reduced by regarding the linear molecular unit as a structure composed of further a plurality of small linear molecular units. When the number of lattice points required for searching for the structure may be reduced, the number of bits required for searching for the structure may be reduced, as described above.

For example, in the technique disclosed herein, in one aspect, by regarding a linear molecular unit as a structure composed of further a plurality of small linear molecular units, lattice points required for searching for a structure may be reduced, so that the number of required bits may be reduced.

In one aspect, the technique disclosed herein may be suitably applied to a molecule having a branched structure. For example, in one example of the technique disclosed herein, a dividing point is a branching point in a molecule having a branched structure, and the molecule is regarded as a structure having a linear molecular unit including from a branching point to a branching end and a linear molecular unit including from one branching point to another branching point adjacent thereto.

In this manner, in one aspect, the technique disclosed herein may search for a stable structure in a molecule having a branched structure while suppressing the number of bits required to search for a molecular structure.

The molecule having a branched structure is not particularly limited and may be appropriately selected according to the purpose, and examples thereof include a protein in a structure including a side chain of an amino acid residue, a polymer having a branched structure used in a soft material field, and the like.

In the following, a protein in a structure including a side chain of an amino acid residue will be described as an example of a molecule having a branched structure.

The technique of related art for searching for a stable structure in the lattice protein searches for structure only in consideration of the structure of the main chain of the protein, and it is not possible to take into consideration of the structure of the side chain of the protein. It is considered that the steric structure of the protein is affected by not only the main chain of the protein but also the structure (position) of the side chain of the amino acid residue forming the protein.

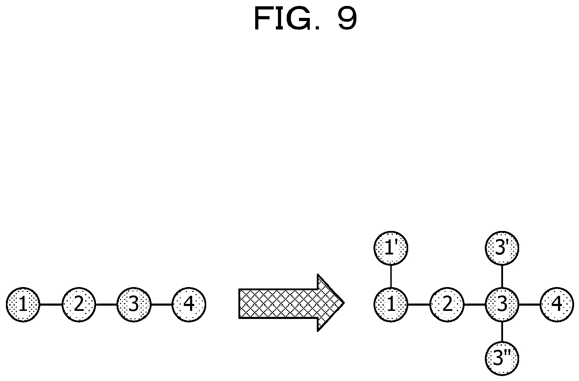

For example, as illustrated in the left part of FIG. 9, in the technique of related art in the lattice protein, an amino acid residue forming a protein as a search target of a structure is treated as one particle of coarse graining, so that the side chain of the amino acid residue is not taken into consideration.

However, as illustrated in the right part of FIG. 9, side chains are present in the actual protein, and in a case where an amino acid residue having an atomic number of 20 or more (a side chain is large) is included in the protein, it is considered that the side chain of the amino acid residue has a large influence on the steric structure of the protein. As illustrated in the right part of FIG. 9, when the side chains of the amino acid residues are taken into consideration in the protein subjected to coarse graining, the protein may be regarded as a molecule having a branched structure.

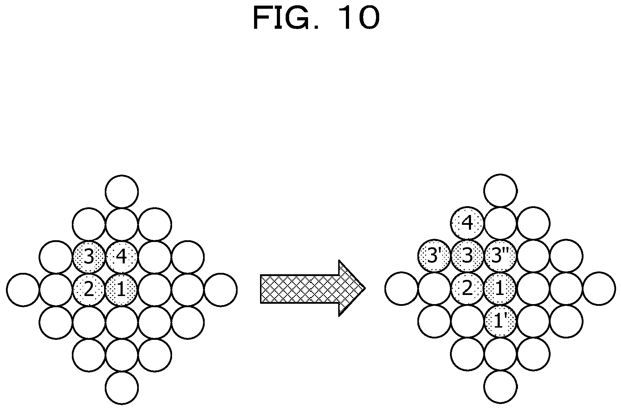

FIG. 10 is a schematic diagram illustrating an example of a case where a stable structure is searched for the molecule illustrated in FIG. 9. As illustrated in the left part of FIG. 10, in the technique of related art in which only the main chain of the protein is taken into consideration, for example, it is assumed that a structure in which the amino acid residue of the number 1 and the amino acid residue of the number 4 interact with each other, and the amino acid residue of the number 1 and the amino acid residue of the number 4 are adjacent to each other is searched for as a stable structure.

However, the stable structure calculated in consideration of the side chain of the amino acid residue forming the protein may differ from the stable structure calculated in consideration of only the main chain of the protein. For example, it is assumed that the interaction between the main chain of the amino acid residue of the number 1 and the side chain 3" of the amino acid residue of the number 3 is larger than the interaction between the amino acid residue of the number 1 and the amino acid residue of the number 4. In this case, for example, as illustrated in the right part of FIG. 10, it is considered that the structure in which the main chain of the amino acid residue of the number 1 and the side chain 3" of the amino acid residue of the number 3 are adjacent to each other is calculated as a stable structure.

In this way, it is possible to obtain a further accurate stable structure of the protein by searching for the stable structure of the protein in consideration of the structure of the side chain of the amino acid residue forming the protein.

In an example of the technique disclosed herein, a dividing point in a protein as a molecule having a branched structure may be a branching point in a molecule having a branched structure, for example, and may be preferably located in a main chain of a protein when the molecule is the protein. In this case, the linear molecular unit becomes a part of the main chain of the amino acid residue or the protein.

For example, a case where an amino acid residue having a side chain equal to or larger than a predetermined size in a main chain of a protein is used as a dividing point will be considered. In this case, the linear molecular unit including from the dividing point (branching point) to a branching end becomes one amino acid residue including the side chain, and the linear molecular unit including from one branching point to another branching point adjacent thereto becomes a part of the main chain of the protein.

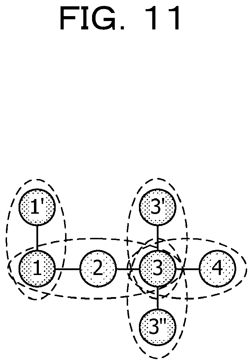

For example, more specifically, as illustrated in FIG. 11, amino acid residues having a side chain having a size equal to or larger than a predetermined size (side chains to be considered) are an amino acid residue of the number 1 and an amino acid residue of the number 3. In this case, the dividing point becomes the amino acid residue of the number 1 and the amino acid residue of the number 3 in the main chain of the protein, and as illustrated in FIG. 11, the protein may be regarded as a structure having 5 linear molecular units.

In this manner, in the technique disclosed herein, in one aspect, the dividing point is located in the main chain of the protein, and the linear molecular unit is a part of the main chain of the amino acid residue or the protein, whereby it is possible to search for a further accurate stable structure in consideration of the structure of the side chain of the amino acid residue.

The amino acid to be the origin of the amino acid residue may be a natural amino acid or an unnatural amino acid.

Examples of the natural amino acid include alanine, arginine, asparagine, aspartic acid, cysteine, glutamine, glutamic acid, glycine, histidine, isoleucine, leucine, lysine, methionine, phenylalanine, proline, serine, threonine, tryptophan, tyrosine, valine, β-alanine, β-phenylalanine, and the like.

Examples of the unnatural amino acid include a chemically modified amino acid residue such as parabenzoyl phenylalanine.

The amino acid residue to be considered for the side chain in the technique disclosed herein is not particularly limited and may be appropriately selected according to the purpose, and for example, an amino acid having 20 or more atoms may be used. For example, more specifically, the amino acid residue to be considered for the side chain may be, for example, an amino acid residue other than glycine and alanine in a case where the molecule is formed of a residue of a natural amino acid. Amino acid residues other than glycine, alanine, and serine may be used depending on conditions for searching for the stable structure of the molecule.

The number of amino acid residues in the protein is not particularly limited and may be appropriately selected according to the purpose, and may be, for example, about 10 or more and about 50 or less, or several 100. In the present embodiment, a molecule having an amino acid residue of about 50 or less is sometimes referred to as a "peptide".

In a case where a stable structure of a molecule of a polymer such as a resin or rubber is searched for, the dividing point may be, for example, an atomic group (for example, a functional group in two sites) or an atom.

In one example of the technique disclosed herein, a stable binding structure of molecules is searched for the steric structure of molecules created by the above-described method. The method of searching for a stable binding structure of a molecule is not particularly limited and may be appropriately selected according to the purpose, but it is preferable to use an annealing method (annealing). For example, in one example of the technique disclosed herein, it is preferable to calculate the steric structure of the molecule having a minimum energy by performing the ground state search using the annealing method for the steric structure of the molecule, which is created by the above-described method. For example, in one aspect, it is preferable that the binding structure search apparatus disclosed herein include a calculation unit for calculating a steric structure of a molecule having a minimum energy by performing the ground state search using the annealing method for the created steric structure of the molecule.

In this manner, in one aspect, the technique disclosed herein may be used to search for the most stable structure of the molecule while suppressing the number of bits required to search for the structure of the molecule. In one aspect, in the technique disclosed herein may be used to further accurate search for the most stable structure of the molecule in consideration of the branched structure even for a molecule having a branched structure, such as a protein having a side chain.

An example of the technique disclosed herein will be described in more detail with reference to a configuration example of the apparatus and a flowchart.

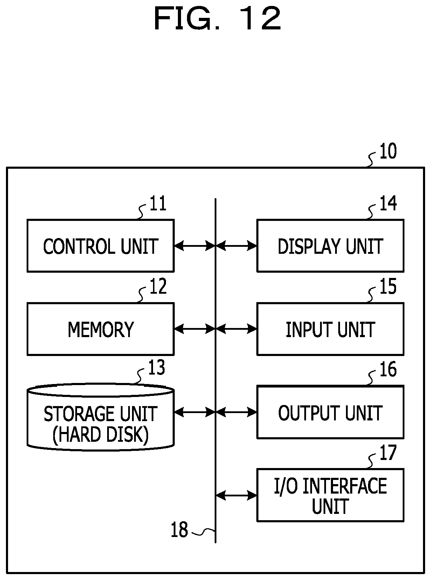

FIG. 12 illustrates a configuration example of a binding structure search apparatus disclosed herein.

In a binding structure search apparatus 10, for example, a control unit 11, a memory 12, a storage unit 13, a display unit 14, an input unit 15, an output unit 16, and an I/O interface unit 17 are coupled via a system bus 18.

The control unit 11 performs operations (four arithmetic operations, comparison operation, operations of annealing method, and the like), operation control of hardware and software, and the like.

The control unit 11 is not particularly limited and may be appropriately selected according to the purpose, and may be, for example, a central processing unit (CPU) or an optimization apparatus used in an annealing method to be described later, and may be a combination thereof.

A creation unit and a calculation unit in the binding structure search apparatus disclosed herein may be realized by, for example, the control unit 11.

The memory 12 is a memory such as a random-access memory (RAM), a read-only memory (ROM), or the like. The RAM stores an operating system (OS), an application program, and the like read from the ROM and the storage unit 13, and functions as a main memory and a work area of the control unit 11.

The storage unit 13 is a device for storing various programs and data, and is a hard disk, for example. The storage unit 13 stores a program to be executed by the control unit 11, data required for execution of the program, the OS, and the like.

The preprocessing program for the binding free energy calculation disclosed herein is stored in the storage unit 13, loaded into the RAM (main memory) of the memory 12, and executed by the control unit 11.

The display unit 14 is a display device, and is, for example, a display device such as a cathode-ray tube (CRT) monitor, or a liquid crystal panel.

The input unit 15 is an input device for various data, and is, for example, a keyboard, a pointing device (for example, a mouse, or the like), or the like.

The output unit 16 is an output device for various data, and is, for example, a printer, or the like.

The I/O interface unit 17 is an interface for coupling various external devices. The I/O interface unit 17 allows input/output of data such as a compact disc read-only memory (CD-ROM), a digital versatile disk read-only memory (DVD-ROM), a magneto-optical (MO) disk, and a USB memory [Universal Serial Bus (USB) flash drive], for example.

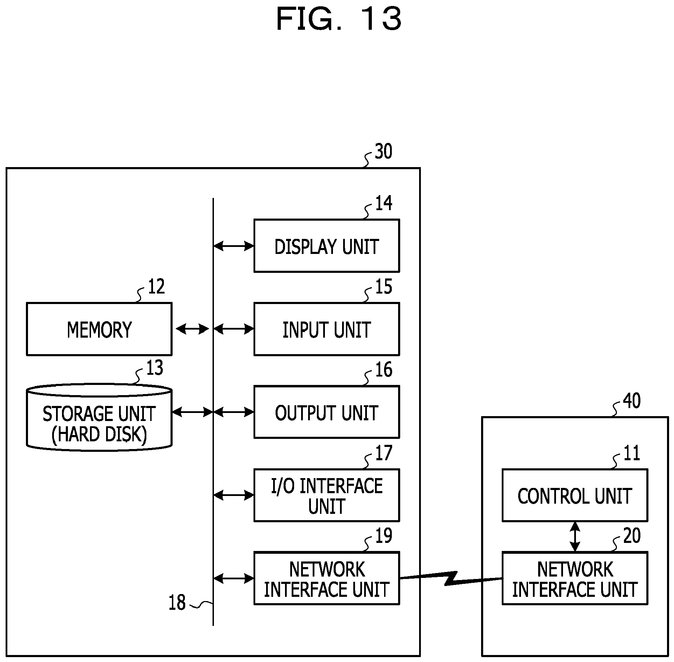

FIG. 13 illustrates another configuration example of the binding structure search apparatus disclosed herein.

The example illustrated in FIG. 13 is an example in which the binding structure search apparatus is a cloud type, and the control unit 11 is independent from the storage unit 13 and the like. In the example illustrated in FIG. 13, a computer 30 in which the storage unit 13 and the like are stored, and a computer 40 in which the control unit 11 is stored are coupled via network interface units 19 and 20.

The network interface units 19 and 20 are hardware configured to perform communication by using the Internet.

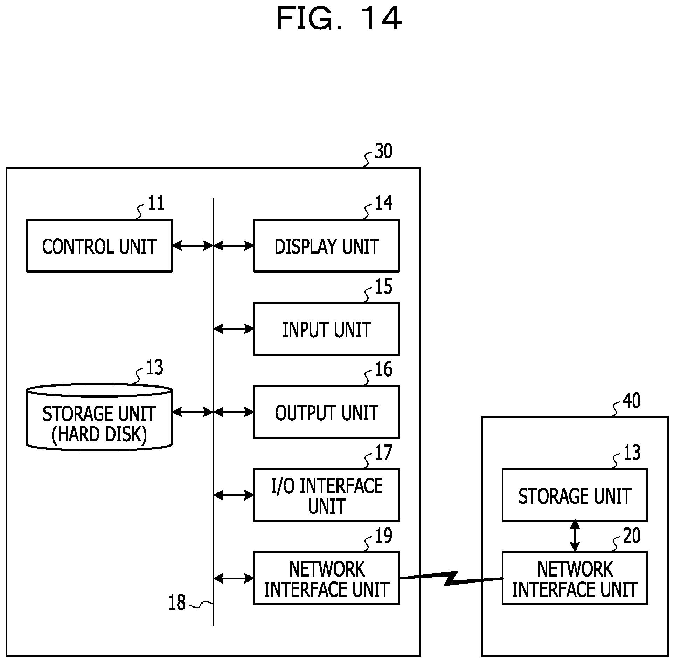

FIG. 14 illustrates another configuration example of the binding structure search apparatus disclosed herein.

The example illustrated in FIG. 14 is an example in which the binding structure search apparatus is a cloud type, and the storage unit 13 is independent from the control unit 11 and the like. In the example illustrated in FIG. 14, the computer 30 in which the control unit 11 and the like are stored, and the computer 40 in which the storage unit 13 is stored are coupled via the network interface units 19 and 20.

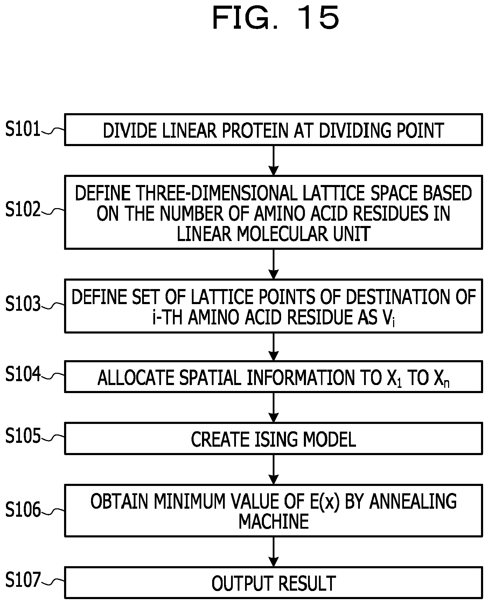

FIG. 15 illustrates an example of a flowchart in searching for a stable structure of a linear protein by using an example of the technique disclosed herein.

First, the control unit 11 divides the protein to be searched for the structure at a dividing point (S101). The position of the dividing point in the protein is not particularly limited and may be appropriately selected according to the purpose, but from the viewpoint of reducing the number of bits required for searching for the structure, it is preferable to use the amino acid residue located near the center of the amino acid sequence of the protein as one of the dividing points. In this example, it is assumed that the number of residues in the protein is n.

Subsequently, according to the number of amino acid residues in the linear molecular unit having the largest number of amino acid residues formed by dividing the protein, a three-dimensional lattice space, which is a set of lattices in which a plurality of amino acid residues is sequentially arranged, is defined (S102).

An example of the definition of the three-dimensional lattice space will now be described. The lattice space is three dimensional, but in the following, a case of two dimensional is taken as an example for simplification.

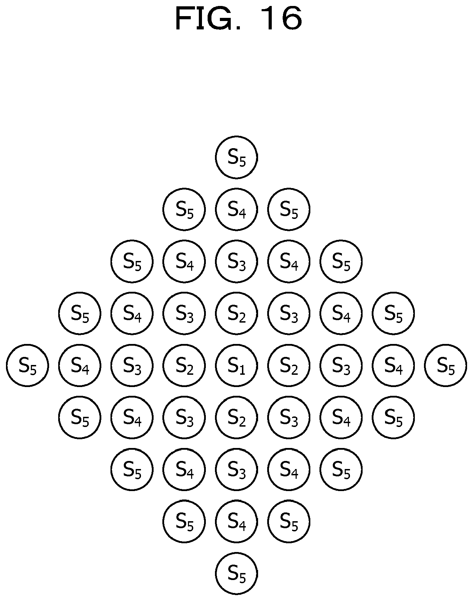

First, a set of lattices having a radius r in a diamond lattice space is referred to as a Shell, and each lattice point is denoted as $S_r$. The lattice points $S_r$ may be represented as illustrated in FIG. 16.

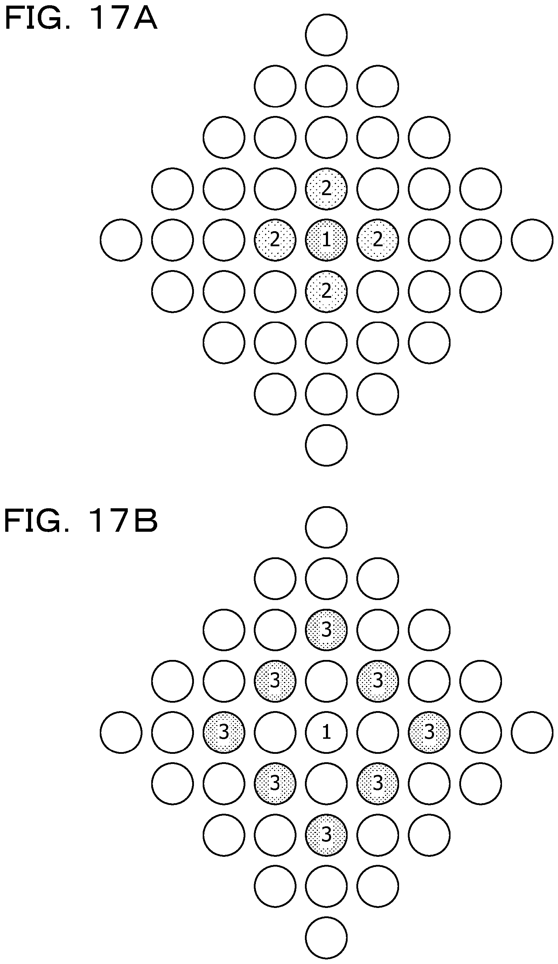

For example, the set $V_1$ to $V_5$ of the lattice points of destinations of the first to fifth amino acid residues becomes as illustrated in FIG. 17A to FIG. 17D.

In FIG. 17A, $V_1 = S_1$, and $V_2 = S_2$.

In FIG. 17B, $V_3 = S_3$.

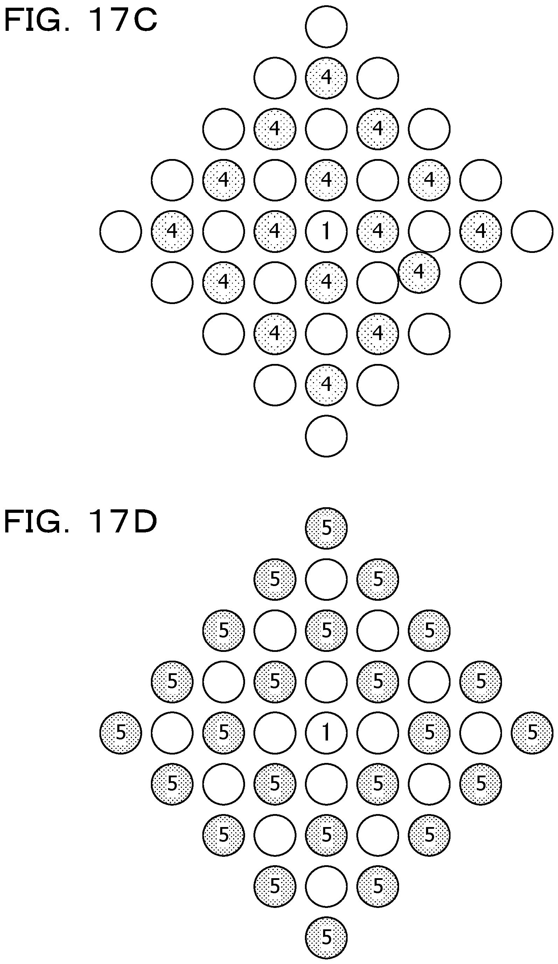

In FIG. 17C, $V_4 = S_2$ or $S_4$.

In FIG. 17D, $V_5 = S_3$ or $S_5$.

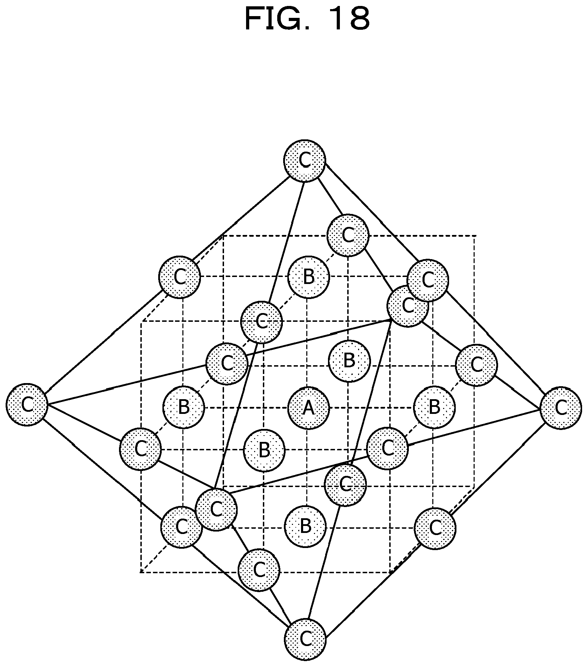

As illustrated in FIG. 18, $S_1$, $S_2$, and $S_3$ are expressed in three dimensions. In FIG. 18, $A = S_1$, $B = S_2$, and $C = S_3$.

A space $V_i$ required for the i-th amino acid residue in the protein having n amino acid residues is represented by the following equation.

$$V_i = \bigcup_{r \in J} S_r$$

Here, $i = \{1, 2, 3, \ldots\ldots n\}$.

In a case of an odd-numbered (i = odd) amino acid residue, $J = \{1, 3, \ldots\ldots i\}$, and in a case of even-numbered (i = even) amino acid residues, $J = \{2, 4, \ldots\ldots i\}$.

Subsequently, the control unit 11 sets the set of lattice points of the destination of the i-th amino acid residue in each linear molecular unit to $V_i$ (S103).

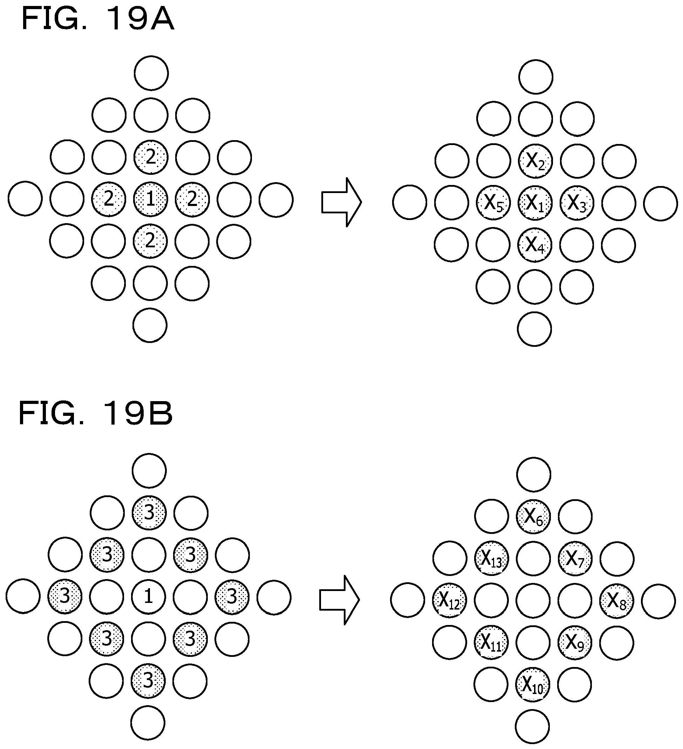

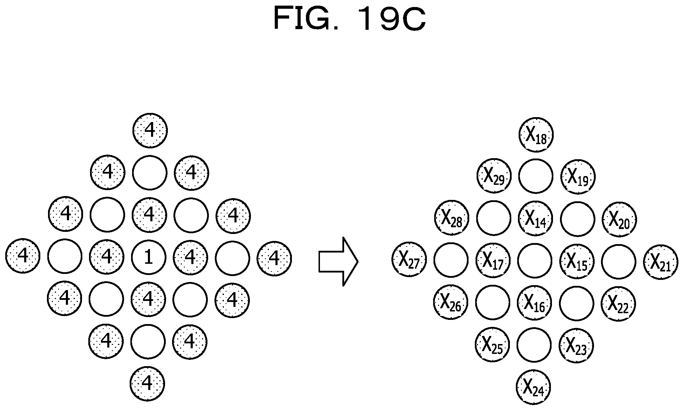

Next, the control unit 11 assigns bits to each lattice point for each of the linear molecular units. For example, information on a space is allocated to each of bits $X_1$ to $X_n$ (S104). For example, as illustrated in FIG. 19A to FIG. 19C, bits are allocated to the space in which each amino acid residue is entered, the bits being represented by "1" with the presence of the amino acid residue at that position and represented by "0" with the absence thereof, respectively. In FIG. 19A to FIG. 19C, a plurality of $X_i$ is assigned to respective amino acid residues 2 to 4, but in practice one bit $X_i$ is assigned to one amino acid residue.

Next, $H_{one}$, $H_{olap}$, $H_{conn1}$, $H_{conn2}$, $H_{pair1}$, and $H_{pair2}$ are set to create an Ising model that is converted based on constraint conditions for each lattice point (S105).

In one example of the technique disclosed herein, the total energy may be expressed as follows:

$$E(x) = H = H_{one} + H_{olap} + H_{conn1} + H_{conn2} + H_{pair1} + H_{pair2}$$

$H_{one}$ represents a constraint that there is only one for each of first to n-th amino acid residues.

$H_{olap}$ represents the constraint that the first to n-th amino acid residues do not overlap with each other.

$H_{conn1}$ represents a constraint that amino acid residues in the same linear molecular unit are coupled to each other so as to satisfy the binding order in the proteins.

$H_{conn2}$ represents a constraint that the linear molecular units are coupled to each other so as to satisfy the binding order in the proteins.

$H_{pair1}$ represents a constraint expressing the interaction between amino acid residues in the same linear molecular unit.

$H_{pair2}$ represents a constraint expressing the interaction between amino acid residues in different linear molecular units.

An example of each constraint is as follows.

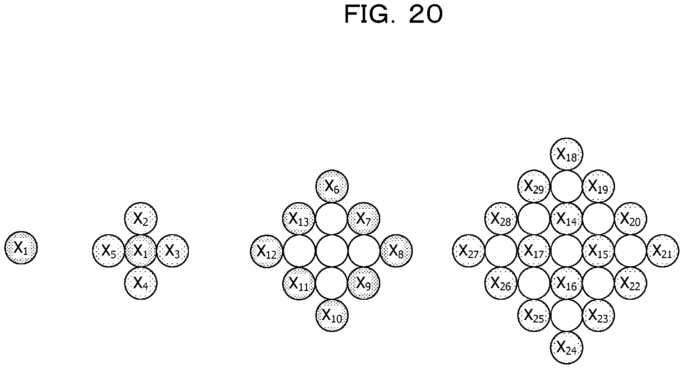

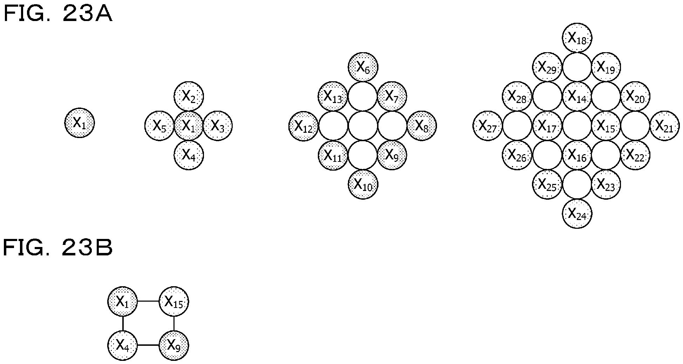

In FIG. 20 to FIG. 23A and FIG. 23B described below, $X_1$ represents a position at which the amino acid residue of the number 1 may be arranged.

$X_2$ to $X_5$ represents a position at which the amino acid residue of the number 2 may be arranged.

$X_6$ to $X_{13}$ represents a position at which the amino acid residue of the number 3 may be arranged.

$X_{14}$ to $X_{29}$ represents a position at which the amino acid residue of the number 4 may be arranged.

An example of $H_{one}$ is indicated below.

$$H_{one} = \lambda_{one} \sum_{i=0}^{N-1} \sum_{x_a, x_b \in Q_i, a<b} x_a x_b$$

In the above function, $X_a$ and $X_b$ take 1 or 0. For example, in FIG. 20, $H_{one}$ is a term of a penalty that becomes 0 in a case where only one of $X_2$, $X_3$, $X_4$, and $X_5$ is 1 in the function in which only any one of them is 1, so that energy is increased in a case where any two or more are 1.

In the above function, $\lambda_{one}$ is a coefficient for weighting.

An example of $H_{olap}$ is indicated below.

$$H_{olap} = \lambda_{olap} \sum_{v \in V} \sum_{x_a, x_b \in \theta(v), a<b} x_a x_b$$

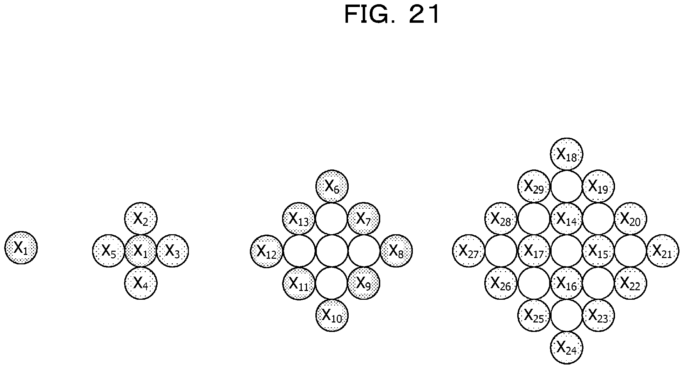

In the above function, $X_a$ and $X_b$ take 1 or 0. For example, in FIG. 21, $H_{olap}$ is a term where a penalty is generated in a case where $X_{14}$ becomes 1 when $X_2$ is 1.

In the above function, $\lambda_{olap}$ is a coefficient for weighting.

An example of $H_{conn1}$ is indicated below.

$$H_{conn1} = \lambda_{conn1}(N - 1 - \sum_{i=0}^{N-1} \sum_{x_d \in Q_i} \sum_{x_u \in \eta(x_d) \cap Q_{i+1}} x_d x_u)$$

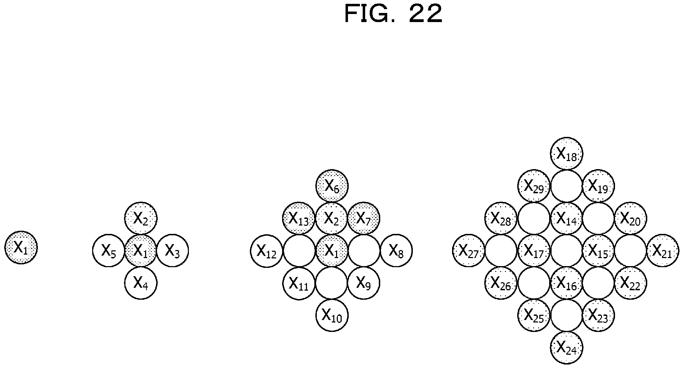

The above function is a function for evaluating a coupling between amino acid residues, and $X_d$ and $X_u$ take 1 or 0. For example, in FIG. 22, when $X_2$ is 1, in the equation in which energy is lowered when any one of $X_{13}$, $X_6$, and $X_7$ is 1, energy is lowered, $H_{conn1}$ is a penalty term that becomes 0 when all amino acid residues in the same linear molecular unit are coupled so as to satisfy the binding order in the protein.

In the above function, $\lambda_{conn1}$ is a coefficient for weighting. For example, the relationship $\lambda_{one} > \lambda_{conn1}$ may be satisfied.

With deformation of the above equation, $H_{conn1}$ may be a function that has a value becoming small and becoming negative when the amino acid residues in the same linear molecular unit are coupled to each other.

An example of $H_{conn2}$ is indicated below.

$$H_{conn2} = \lambda_{conn2}(N - 1 - \sum_{i=0}^{N-1} \sum_{x_d \in Q_i} \sum_{x_u \in \eta(x_d) \cap Q_{i+1}} x_d x_u)$$

The above function is a function for evaluating a coupling between the linear molecular units, and $X_d$ and $X_u$ take 1 or 0. For example, in FIG. 22, when $X_2$ is 1, in the equation in which energy is lowered when any one of $X_{13}$, $X_6$, and $X_7$ is 1, $H_{conn2}$ is a penalty term that becomes 0 when all of the linear molecular units are coupled so as to satisfy the binding order in the protein.

In the above function, $\lambda_{conn2}$ is a coefficient for weighting.

Moreover, $H_{conn2}$ may be a function such that when the above equation is modified, the values become smaller and become negative when the linear molecular units are coupled to each other.

An example of the $H_{pair1}$ is indicated below.

$$H_{pair1} = \frac{1}{2} \sum_{i=0}^{N-1} \sum_{x_a \in Q_i} \sum_{x_b \in \eta(x_a)} P_{\omega(x_a)\omega(x_b)} x_a x_b$$

In the above function, $X_a$ and $X_b$ take 1 or 0. For example, in FIG. 23A and FIG. 23B, when $X_1$ is 1 for the amino acid residue of the same linear molecular unit, $H_{pair1}$ is a function in which the interaction $P_{\omega(x1)\omega(x15)}$ between the amino acid residue of $X_1$ and the amino acid residue $X_{15}$ acts to decrease energy in a case where $X_{15}$ becomes 1. The interaction $P_{\omega(x1)\omega(x15)}$ is determined by the combination of two amino acid residues, and the interaction $P_{\omega(x1)\omega(x15)}$ is determined with reference to, for example, Miyazawa-Jernigan (MJ) matrix. In a case where the protein as a search target of a structure includes unnatural amino acid residue, the interaction parameter between the unnatural amino acid residue and the other amino acid residue is suitably created and used.

An example of $H_{pair2}$ is indicated below.

$$H_{pair2} = \frac{1}{2} \sum_{i=0}^{N-1} \sum_{x_a \in Q_i} \sum_{x_b \in \eta(x_a)} P_{\omega(x_a)\omega(x_b)} x_a x_b$$

In the above function, $X_a$ and $X_b$ take 1 or 0. For example, in FIGs. 23A and 23B, when $X_1$ is 1 for amino acid residues of different linear molecular units, $H_{pair2}$ is a function in which the interaction $P_{\omega(x1)\omega(x15)}$ between the amino acid residue of $X_1$ and the amino acid residue of $X_{15}$ acts to decrease energy in a case where $X_{15}$ becomes 1. The interaction $P_{\omega(x1)\omega(x15)}$ is determined by the combination of two amino acid residues, and the interaction $P_{\omega(x1)\omega(x15)}$ is determined with reference to, for example, Miyazawa-Jernigan (MJ) matrix. In a case where the protein as a search target of a structure includes unnatural amino acid residue, the interaction parameter between the unnatural amino acid residue and the other amino acid residue is suitably created and used.

H is calculated by synthesizing Hone, $H_{olap}$, $H_{conn1}$, $H_{conn2}$, $H_{pair1}$, and $H_{pair2}$.

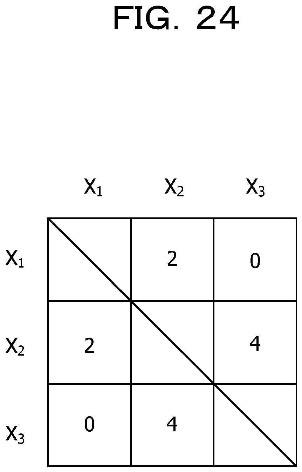

Next, a weight file corresponding to a weight coefficient (for example, $\lambda_{one}$, $\lambda_{olap}$, $\lambda_{conn1}$, $\lambda_{conn2}$, or the like) in the above functions extracted and optimized through the calculation using the energy equation of the following Ising model is, for example, a matrix, and is a file of the matrix as illustrated in FIG. 24 in a case of $2X_1X_2 + 4X_2X_3$.

$$E(x) = -\sum_{(i,j)} W_{ij} x_i x_j - \sum_i b_i x_i$$

In the above function, the states $X_i$ and $X_j$ are "0" or "1", and "0" means that there is no amino acid residue, and "1" means that an amino acid residue is present. $W_{ij}$ of a first term on a right side is a coefficient for weighting.

The first term on the right side represents the sum of products of states of two circuits and a weight value without missing or redundantly counting for all combinations of two circuits selectable from all circuits.

A second term on the right side represents the sum of products of individual bias values and the state of all the circuits. $b_i$ indicates a bias value of an i-th circuit.

A description will be given of a method of searching for a stable binding structure of molecules having a branched structure by using the energy equation of the above Ising model.

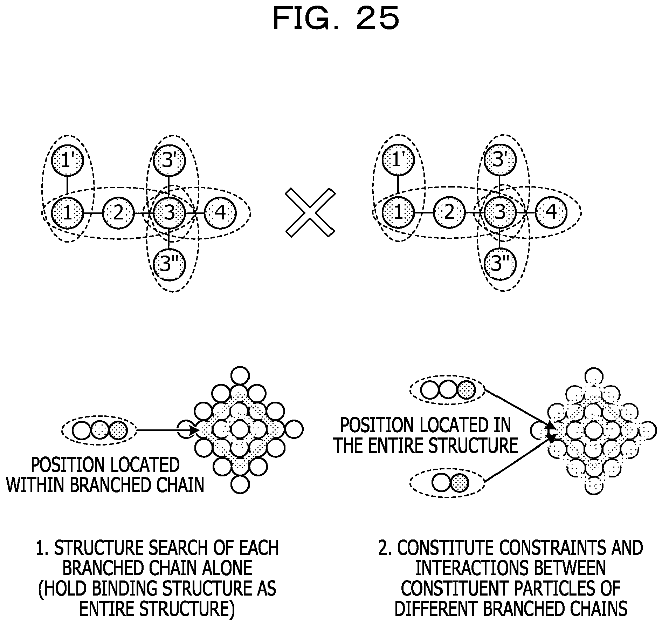

The energy equation of the above Ising model may be considered to be a combination of a Hamiltonian in a case where each linear molecular unit (branched chain) is regarded as the main chain structure in the related art, and a Hamiltonian in which a constraint and interaction between the branched chains are taken into account. For example, for the particles within the branched chain, a Hamiltonian that represents the constraint and interaction with which a binding order between particles may be maintained when viewed from the entire molecule, and for the particles between respective branched chains, a Hamiltonian that represents the constraint and interaction with which the binding order of molecules is maintained, are combined with each other. As illustrated in FIG. 25, this is the same calculation as to obtain a direct product of the condition within the branched chain and the condition between the branched chains.

By searching for the positions of the particles that reduce the energy equation of the above Ising model reflecting the above conditions, it is possible to search for stable structures with no contradiction as a molecular structure. For example, by searching for the positions of the particles that reduce the energy equation of the above Ising model, for the linear molecular units including the same dividing point, it is possible to arrange the linear molecular units so as not to overlap with each other and also arrange it in a manner such that the same dividing points are located at the same lattice point.

Next, in an annealing machine, a ground state search using an annealing method is performed on an Ising model converted based on a constraint condition for each lattice point, thereby calculating a minimum energy of the Ising model (S106).

The annealing machine is not particularly limited as long as it is a computer employing an annealing method for performing the ground state search on an energy function represented by the Ising model, and may be appropriately selected according to the purpose. Examples of the annealing machine include a quantum annealing machine, a semiconductor annealing machine using a semiconductor technology, and a machine for performing simulated annealing performed by software using a CPU or a graphics processing unit (GPU). As an annealing machine, for example, Digital Annealer (registered trademark) may be used.

In S107, the calculation result is output. The result may be output as a steric structure diagram of the protein or as coordinate information of each amino acid residue configuring the protein.

In this way, by searching for a stable structure of a protein, it is possible to search for a structure of the protein, which is considered to be most stable, while suppressing the number of bits required for searching for the structure of the molecule.

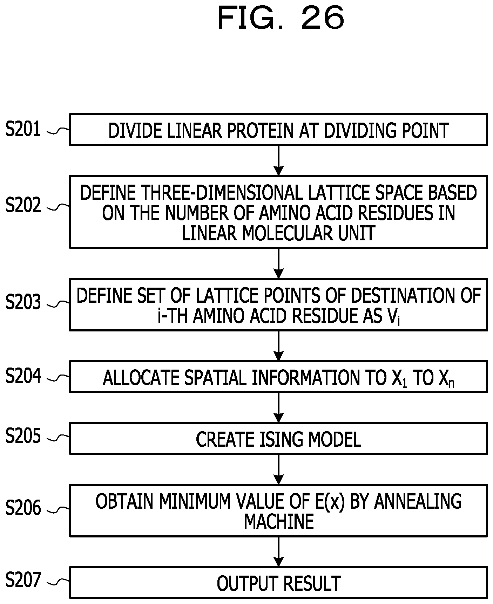

FIG. 26 illustrates an example of a flowchart in searching for a stable structure of a protein in consideration of the structure of a side chain by using an example of the technique disclosed herein.

In FIG. 26, since steps S202 to S207 are similar to steps S102 to S107 in FIG. 15, thus the description thereof will not be repeated.

In S201, the control unit 11 divides the protein to be searched for the structure by using the amino acid residue to be taken into consideration of the structure of the side chain in the main chain of the protein as the dividing point. As the amino acid residue to be considered for the structure of the side chain, as described above, for example, an amino acid residue other than glycine and alanine may be used.

In this manner, by dividing the protein to be regarded as a structure having a plurality of linear molecular units, it is possible to further accurate search for a structure of the protein which is considered to be most stable in consideration of the structure of the side chain.

An example of an annealing method and an annealing machine will be described below.

The annealing method is a method of obtaining a solution stochastically by using a random number value or a superposition of quantum bits. Hereinafter, an object of minimizing a value of an evaluation function to be optimized will be described as an example, and the value of the evaluation function will be referred to as energy. When the value of the evaluation function is maximized, a sign of the evaluation function may be changed.

First, starting with an initial state in which one discrete value is assigned to an individual variable, from the current state (a combination of values of variables), a state close to the current state (for example, a state in which only one of the variables has been changed) is selected, and this state transition is examined. A change in energy associated with the state transition is calculated, and it is stochastically determined whether to adopt the state transition and change the current state or to maintain the original state without adopting the state transition, according to the calculated value. When setting an adoption probability of a state transition that results in a drop in the energy to be greater than that of a state transition that results in a rise in the energy, state changes occur in a direction in which the energy drops on average, and thus it is possible to expect that the state is transitioned to a more suitable state with the lapse of time. Therefore, an approximate solution that possibly results in energy close to the optimal solution or optimal value may be finally obtained.

When a state transition that results in a drop in the energy in a deterministic way is adopted and a state transition that results in a rise in the energy is not adopted, the change in energy broadly monotonically decreases over time, however, once a local solution is reached, no further change may occur. Since an extraordinarily large number of local solutions exist in a discrete optimization problem as described above, the state is stuck at a local solution that is not very close to an optimal value, in many cases. Therefore, in solving a discrete optimization problem, it is important to determine whether or not to adopt the state stochastically.

In the annealing method, it has been proved that the state reaches the optimal solution at a limit of infinite time (the number of iterations) as long as the adoption (acceptance) probability of the state transition is determined as follows.

Hereinafter, a method for determining an optimal solution using an annealing method will be described in order.

(1) For an energy change (energy decrease) value ($-\Delta E$) associated with a state transition, an acceptance probability p of the state transition is determined by any of the following functions f( ).

$$p(\Delta E, T) = f(-\Delta E / T) \quad (1\text{-}1)$$

$$f_{metro}(x) = \min(1, e^x) \qquad \text{(Metropolis Method)}(1\text{-}2)$$

$$f_{Gibbs}(x) = \frac{1}{1 + e^{-x}} \qquad \text{(Gibbs Method)}(1\text{-}3)$$

Here, T is a parameter called a temperature value, and for example, may be changed as follows.
(2) A temperature value T is logarithmically reduced with respect to the number of iterations t as represented by the following equation.

$$T = \frac{T_0 \log(c)}{\log(t + c)} \quad (2)$$

Here, $T_0$ represents an initial temperature value and it is desirable that a sufficiently large value be set in accordance with the problem.

In a case of using the acceptance probability expressed by Equation (1), when a steady state is reached after sufficient number of iterations, an occupation probability of an individual state is in accordance with a Boltzmann distribution at thermal equilibrium state in thermodynamics.

Since the occupation probability of a lower-energy state increases when the temperature gradually decreases from high initial temperature, a low-energy state is supposed to be obtained when the temperature has sufficiently decreases. This method is referred to as an annealing method (or pseudo-annealing method) because this behavior resembles state change when annealing a material. The stochastic occurrence of a state transition that results in a rise in the energy corresponds to thermal excitation in physics.

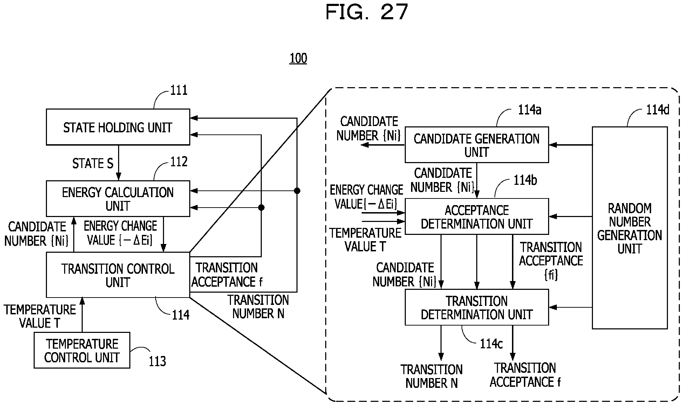

FIG. 27 illustrates an example of a functional configuration of an optimization apparatus (control unit 11) for performing the annealing method. While, cases where a plurality of candidates for the state transition is generated will be also described in the following description, the transition candidates are generated one by one in the basic annealing method.

An optimization apparatus 100 includes a state holding unit 111 configured to hold a current state S (values of a plurality of state variables). The optimization apparatus 100 also includes an energy calculation unit 112 configured to calculate energy change values {-ΔEi} of state transitions in a case where the state transition occurs from the current state S as a result of change in any of the values of the plurality of state variables. The optimization apparatus 100 includes a temperature control unit 113 configured to control the temperature value T and a transition control unit 114 configured to control state changes.

The transition control unit 114 stochastically determines whether or not any one of a plurality of state transitions is accepted, depending on a relative relationship between the energy change values {-ΔEi} and thermal excitation energy based on the temperature value T, the energy change values {-ΔEi}, and the random number value.

The transition control unit 114 includes a candidate generation unit 114a for generating a candidate for a state transition, and an acceptance determination unit 114b for stochastically determining whether or not the state transition is accepted from the energy change values {-ΔEi} of the candidates and the temperature value T for each candidate. The transition control unit 114 includes a transition determination unit 114c for determining a candidate to be adopted from the accepted candidates, and a random number generation unit 114d for generating a probability variable.

The operation in one iteration in the optimization apparatus 100 is as follows.

First, the candidate generation unit 114a generates one or a plurality of candidates (candidate numbers {Ni}) for the state transition from the current state S held by the state holding unit 111 to the next state. The energy calculation unit 112 calculates energy change values {-ΔEi} for each of the state transitions for the candidates, by using the current state S and the candidates for the state transition. The acceptance determination unit 114b uses the temperature value T generated in the temperature control unit 113 and a probability variable (random number value) generated by the random number generation unit 114d, and accepts the state transition with the acceptance probability expressed by the above Equation (1) according to the energy change values {-ΔEi} of the respective state transitions.

Then, the acceptance determination unit 114b outputs the acceptances {fi} of the respective state transitions. In a case where a plurality of state transitions is accepted, the transition determination unit 114c randomly selects one thereof by using a random number value. The transition determination unit 114c then outputs a transition number N of the selected state transition, and a transition acceptance f. In a case where there is an accepted state transition, the values of the state variable stored in the state holding unit 111 is updated according to the adopted state transition.

Starting with the initial state, the above-described iteration processes are repeated while causing the temperature control unit 113 to lower the temperature value, and the operation ends when a certain number of iterations is reached, or when an end determination condition, for example, the energy becomes lower than a predetermined value, is satisfied. The solution outputted by the optimization apparatus 100 is the state corresponding to the end of the operation.

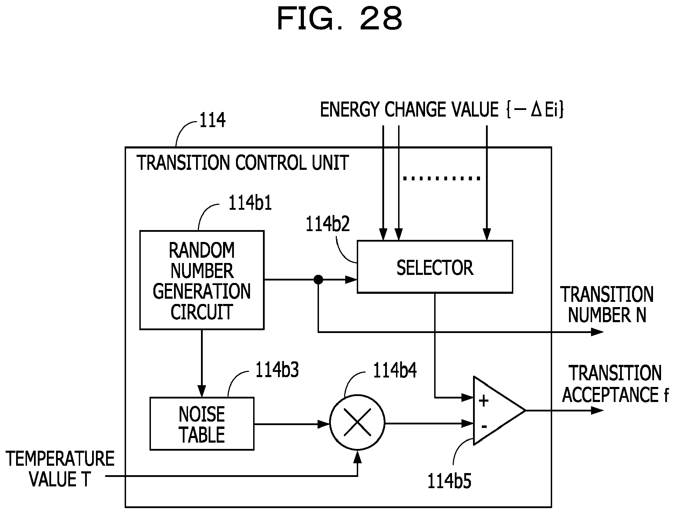

FIG. 28 is a block diagram of a transition control unit in a normal annealing method for generating candidates one by one, for example, a block diagram of a circuit level of a configuration example of an arithmetic portion required for the acceptance determination unit.

The transition control unit 114 includes a random number generation circuit 114b1, a selector 114b2, a noise table 114b3, a multiplier 114b4, and a comparator 114b5.

Of all the energy change values {-ΔEi} calculated for the candidates of the respective state transitions, the selector 114b2 selects and outputs an energy change value corresponding to the transition number N, which is a random number value generated by the random number generation circuit 114b1.

Functions of the noise table 114b3 will be described later. As the noise table 114b3, for example, a memory such as a RAM, a flash memory, or the like may be used.

The multiplier 114b4 outputs a product obtained by multiplying a value outputted by the noise table 114b3 by the temperature value T (corresponding to the thermal excitation energy described above).

The comparator 114b5 outputs a comparison result in which the multiplication result outputted by the multiplier 114b4 is compared with the energy change value -ΔE that is the energy change value selected by the selector 114b2, as the transition acceptance f.

Although the transition control unit 114 illustrated in FIG. 28 basically implements the functions described above without change, a mechanism of accepting a state transition with the acceptance probability expressed by Equation (1) will be described in more detail.

A circuit that outputs 1 when the acceptance probability p is established and outputs 0 when the acceptance probability (1-p) is established may be realized by a comparator that has two inputs A and B, outputs 1 when A > B, and outputs 0 when A < B by inputting the acceptance probability p to the input A and a uniform random number having a value in a section [0, 1) to the input B. Thus, with an input of the value of the acceptance probability p calculated by using Expression (1) based on the energy change value and the temperature value T to the input A of the comparator, it is possible to realize the above function.

For example, assuming that f is the function used in Expression (1), and that u is a uniform random number having a value in the section [0, 1), the circuit that outputs 1 when f(ΔE/T) is greater than u realizes the above function.

The same function as that described above may be realized by any of the following variations.

Even when the same monotonically increasing function is applied to two numbers, the two numbers maintain the same magnitude relationship. Therefore, even when the same monotonically increasing function is applied to the two inputs of the comparator, the same output is obtained. When an inverse function $f^{-1}$ of f is adopted as this monotonically increasing function, it is seen that a circuit that outputs 1 when -ΔE/T is greater than $f^{-1}(u)$ may be adopted. Since the temperature value T is positive, it is seen that a circuit that outputs 1 when -ΔE is greater than $Tf^{-1}(u)$ is suitable.

The noise table 114b3 in FIG. 28 is a conversion table for realizing the inverse function $f^{-1}(u)$, and is a table for outputting a value of the next function with respect to the input obtained by discretizing the section [0, 1).

$$f^{-1}_{metro}(u) = \log(u) \quad \text{(3-1)}$$

$$f^{-1}_{Gibbs}(u) = \log\left(\frac{u}{1-u}\right) \quad \text{(3-2)}$$

Although the transition control unit 114 includes a latch that holds a determination result and the like, a state machine that generates the corresponding timing, and the like, these components are not illustrated in FIG. 28 for simple illustration.

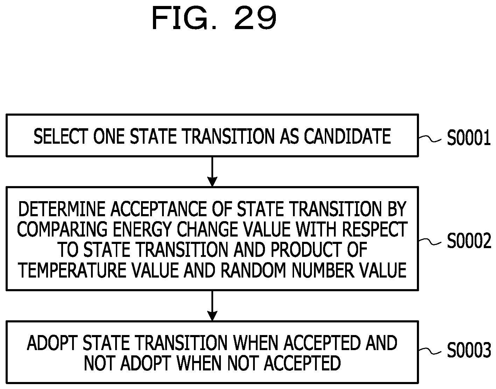

FIG. 29 is a diagram illustrating an example of an operation flow of the transition control unit 114. The operation flow illustrated in FIG. 29 includes a step of selecting one state transition as a candidate (S0001), a step of determining whether a state transition is accepted or not by comparing the energy change value with respect to the state transition with a product of a temperature value and a random number value (S0002), and a step (S0003) in which the state transition is adopted when the state transition is accepted, and the state transition is not adopted when the state transition is not accepted.

(Binding Structure Search Method)

The binding structure search method disclosed herein is a method for searching for a stable binding structure of molecules by using a computer, and includes: dividing a molecule by at least one dividing point and regarding the molecule as a structure composed of one linear molecular unit including one dividing point and another linear molecular unit including one dividing point; arranging one linear molecular unit and the other linear molecular unit at each lattice point of a three-dimensional lattice space that is a set of lattices, arranging the linear molecular unit having same dividing points so as not to overlap with each other and also arranging in a manner such that the same dividing points are located at the same lattice point; and creating a steric structure of the molecule in the three-dimensional lattice space.

The binding structure search method disclosed herein may be performed by, for example, a binding structure search apparatus disclosed herein. Further, a preferred embodiment of the binding structure search method disclosed herein may be the same as the preferred embodiment of the binding structure search apparatus disclosed herein.

(Binding Structure Search Program)

The binding structure search program disclosed herein is a program for searching for a stable binding structure of a molecule, and causes a computer to perform processes of: dividing the molecule at at least one dividing point and regarding the molecule as a structure composed of one linear molecular unit including one dividing point and another linear molecular unit including one dividing point; arranging one linear molecular unit and another linear molecular unit at each lattice point of a three-dimensional lattice space that is a set of lattices; arranging the linear molecular unit having same dividing points so as not to overlap with each other and also arranging it in a manner such that the same dividing points are located at the same lattice point; and creating a steric structure of the molecule in the three-dimensional lattice space.

The binding structure search program disclosed herein may be, for example, a program that causes a computer to execute a binding structure search method as disclosed herein. The preferred embodiment of the binding structure search program disclosed herein may be the same as the preferred embodiment of the binding structure search apparatus disclosed herein.

The binding structure search program disclosed herein may be created using any of various known program languages according to a configuration of a computer system to be used, and a type, a version, and the like of an operating system.

The binding structure search program disclosed herein may be recorded on a recording medium such as a built-in hard disk, an external hard disk, or the like, or recorded on a recording medium such as a CD-ROM, a DVD-ROM, an MO disk, or a USB memory.

In a case where the binding structure search program disclosed herein is recorded on the recording medium described above, the binding structure search program is directly used, or used by installing the binding structure search program on a hard disk, through a recording medium reading apparatus included in the computer system, as required. The binding structure search program disclosed herein may be recorded in an external storage area (another computer or the like) accessible from the computer system through an information communication network. In this case, the binding structure search program disclosed herein, which is recorded in the external storage area may be directly used or be used by installing the binding structure search program on the hard disk from the external storage area through the information communication network, as required.

The binding structure search program disclosed herein may be divided and recorded on a plurality of recording media for each arbitrary process.

(Computer Readable Recording Medium)

The computer readable recording medium disclosed herein is configured to record the binding structure search program disclosed herein.

The computer readable recording medium disclosed herein is not particularly limited, and may be appropriately selected according to the purpose, and examples thereof include, for example, a built-in hard disk, an external hard disk, a CD-ROM, a DVD-ROM, an MO disk, a USB memory, and the like.

The computer readable recording medium disclosed herein may be a plurality of recording media in which the binding structure search program disclosed herein is divided and recorded for each arbitrary process.

<Embodiment 1>

As one embodiment of the binding structure search apparatus disclosed herein, an example of searching for a stable binding structure for a peptide (hereinafter referred to as a peptide 1) of an amino acid sequence AAAAA ("A" means alanine) will be described. In Embodiment 1, it is assumed that the structure of a side chain is not taken into consideration for the alanine that is an amino acid residue having the side chain composed only of a hydrogen atom.

First, in Embodiment 1, coarse graining for the peptide 1 is performed with each of the amino acid residues as one particle. Next, the peptide 1 is divided with the amino acid residue located in the center of the sequence (the third alanine residue from an end in a case of the peptide 1) as a dividing point, and the peptide 1 is regarded as a structure having two linear molecular units. The potential for defining the interaction between amino acid residues is determined by reference to Miyazawa-Jernigan (MJ) matrix described above.

Subsequently, a Hamiltonian of the quadratic unconstrained binary optimization (QUBO) expression is generated based on such as the constraint that the linear molecular units in the peptide 1 may be arranged so as not to overlap with each other and also arranged in a manner such that the dividing points are located at the same lattice point, and on the interaction between the amino acid residues.

The annealing machine searches for the structure of the peptide 1 in which the generated value of the Hamiltonian becomes minimum.

In this way, in the related art, the entire space in which five amino acids may be arranged to be taken into consideration, whereas in the example of Embodiment 1, by searching only for the space in which three amino acids may be arranged, the stable structure of the peptide 1 may be searched for. Therefore, in the example of Embodiment 1, the number of bits required in the annealing machine may be reduced to 1/3 or less of the number of bits required in the related art, and the stable structure may be efficiently searched for.

<Embodiment 2>

As one embodiment of the binding structure search apparatus disclosed herein, an example of a peptide (hereinafter referred to as a peptide 2) in which the amino acid sequence AK (K') AA ("K" means lysine and "K"' means a side chain of a lysine residue) is searched for a stable binding structure is described. In Embodiment 2, the structure of the peptide 2 is searched in consideration of the structure of the side chain of the lysine residue.

First, in Embodiment 2, the side chains of each of the amino acid residues and lysine residues are subjected to coarse graining as one particle for the peptide 2. Next, the peptide 2 is divided with a lysine residue in a main chain of the peptide 2 as a dividing point, which is a branching point of the peptide 2, and the peptide 2 is regarded as a structure having three linear molecular units. The potential for defining the interaction between the amino acid residues is determined by reference to Miyazawa-Jernigan (MJ) matrix described above.

Subsequently, the Hamiltonian of the QUBO expression is generated based on such as the constraint that the linear molecular units in the peptide 2 may be arranged so as not to overlap with each other and also arranged in a manner such that the dividing points are located at the same lattice point, and on the interaction between the amino acid residues.

The annealing machine searches for the structure of the peptide 2 in which the generated value of the Hamiltonian becomes minimum.

By doing so, the stable structure may be searched in consideration of the structure of the side chain of the amino acid residue forming the peptide 2, so that the further accurate stable structure of the protein may be efficiently obtained.

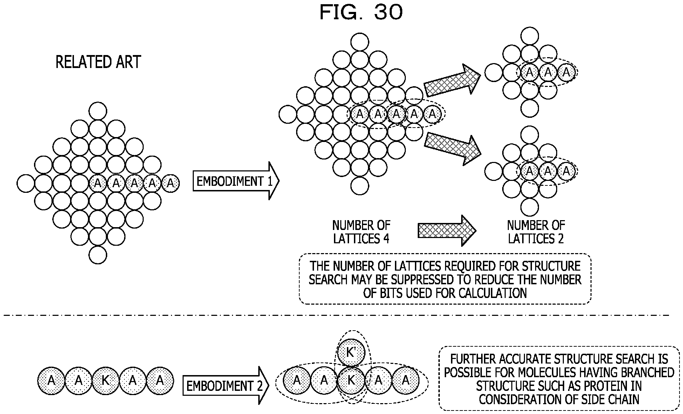

FIG. 30 is a diagram illustrating an example of the number of lattice points required in searching for stable binding structures of proteins (peptides) and the difference between the searched stable structures in Embodiments 1 and 2, and in the related art.

As illustrated in FIG. 30, in one aspect, the technique disclosed herein may reduce the number of bits used when searching for a stable binding structure of a molecule by a calculator in the example of Embodiment 1. Furthermore, in the technique disclosed herein, in one aspect, it is possible to efficiently calculate a further accurate stable structure of a protein for a molecule having a branched structure such as a protein in a case where side chains are taken into consideration in the example of Embodiment 2.

**Claims**

1.  A binding structure search apparatus configured to search for a stable binding structure of a molecule, comprising a creation unit,
    wherein the molecule is divided at at least one dividing point, and is regarded as a structure having one linear molecular unit including the one dividing point and another linear molecular unit including the one dividing point,
    the linear molecular unit and the other linear molecular units are arranged at each lattice point of a three-dimensional lattice space that is a set of lattices,
    the linear molecular units including same dividing points are arranged so as not to overlap with each other, and also arranged in a manner such that the same dividing points are located at the same lattice point, and
    the creation unit is configured to create a steric structure of the molecule in the three-dimensional lattice space.

2.  The binding structure search apparatus according to claim 1,
    wherein the molecule is a molecule having a branched structure,
    the dividing point is a branching point in a molecule having the branched structure, and
    a molecule having the branched structure is regarded as a structure having a linear molecular unit including from the branching point to a branching end, and a linear molecular unit including from one branching point to adjacent another branching point.

3.  The binding structure search apparatus according to any of claims 1 or 2, wherein the linear molecular unit is regarded as a structure composed of further a plurality of small linear molecular units.

4.  The binding structure search apparatus according to any of claims 1 or 2, further comprising a calculation unit configured to calculate a steric structure of the molecule having a minimum energy by performing a ground state search using an annealing method, for a created steric structure of the molecule.

5.  The binding structure search apparatus according to any of claims 1 or 2, wherein the molecule is a protein.

6.  The binding structure search apparatus according to claim 5, wherein the dividing point is located in a main chain of the protein, and the linear molecular unit is an amino acid residue or a part of a main chain of the protein.

7.  A binding structure search method that is a method of searching for a stable binding structure of a molecule using a computer, the method comprising,
    dividing the molecule at at least one dividing point, and regarding as a structure composed of one linear molecular unit including the one dividing point and another linear molecular unit including the one dividing point,
    arranging the linear molecular unit and the other linear molecular units at each lattice point of a three-dimensional lattice space that is a set of lattices,
    arranging the linear molecular units including same dividing points so as not to overlap with each other, and also arranging in a manner such that the same dividing points are located at the same lattice point, and
    creating the steric structure of the molecule in the three-dimensional lattice space.

8.  A program that causes a computer to execute a process for searching a stable binding structure of a molecule, the process comprising:

    dividing the molecule at at least one dividing point, and regarding as a structure composed of one linear molecular unit including the one dividing point and another linear molecular unit including the one dividing point,
    arranging the linear molecular unit and the other linear molecular units at each lattice point of a three-dimensional lattice space that is a set of lattices,
    arranging the linear molecular unit including same dividing points so as not to overlap with each other, and also arranging the linear molecular unit in a manner such that the same dividing points are located at the same lattice point; and
    creating a steric structure of the molecule in the three-dimensional lattice space.

9.  The program according to claim 8, wherein
    the molecule is a molecule having a branched structure,
    the dividing point is a branching point in a molecule having the branched structure, and
    the regarding includes regarding a molecule having the branched structure as a structure having a linear molecular unit including from the branching point to a branching end, and a linear molecular unit including from one branching point to adjacent another branching point.

10. The program according to claim 8,
   wherein the regarding includes regarding the linear molecular unit as a structure composed of further a plurality of small linear molecular units.

11. The program according to claim 8, wherein the process further comprising
   calculating a steric structure of the molecule having a minimum energy by performing a ground state search using an annealing method, for a created steric structure of the molecule.

12. The program medium according to claim 8,
   wherein the molecule is a protein.

13. The program medium according to claim 12,
   wherein the dividing point is located in a main chain of the protein, and
   the linear molecular unit is an amino acid residue or a part of a main chain of the protein.

FIG. 1A

FIG. 1B

FIG. 1C

FIG. 2A

FIG. 2B

FIG. 2C

# FIG. 2D

# FIG. 2E

# FIG. 4

RADIUS=5

# FIG. 5

RADIUS=3

# FIG. 6

# FIG. 7

# FIG. 8

# FIG. 9

# FIG. 10

# FIG. 11

# FIG. 12

10

CONTROL UNIT — 11

DISPLAY UNIT — 14

MEMORY — 12

INPUT UNIT — 15

STORAGE UNIT (HARD DISK) — 13

OUTPUT UNIT — 16

I/O INTERFACE UNIT — 17

18

# FIG. 13

# FIG. 14

```
                                    ┌─30
┌──────────────────────────────────────────┐
│                                            │
│   ┌─11                  ┌─14                │
│   ┌──────────────┐      ┌──────────────┐   │
│   │ CONTROL UNIT │◄────►│ DISPLAY UNIT │   │
│   └──────────────┘      └──────────────┘   │
│                          ┌─15              │
│                         ┌──────────────┐   │
│                    ◄────►│  INPUT UNIT  │   │
│                         └──────────────┘   │
│   ┌─13                   ┌─16              │
│   ┌──────────────┐      ┌──────────────┐   │
│   │ STORAGE UNIT │◄────►│ OUTPUT UNIT  │   │
│   │ (HARD DISK)  │      └──────────────┘   │
│   └──────────────┘       ┌─17             │
│                         ┌──────────────┐   │
│                    ◄────►│I/O INTERFACE │   │
│                         │    UNIT       │   │
│                         └──────────────┘   │
│                          ┌─19              │
│                         ┌──────────────┐   │
│                    ◄────►│   NETWORK    │   │
│   18 ┐                  │INTERFACE UNIT │   │
│                         └──────────────┘   │
└──────────────────────────────────────────┘
```

FIG. 14

# FIG. 15

S101 — DIVIDE LINEAR PROTEIN AT DIVIDING POINT

S102 — DEFINE THREE-DIMENSIONAL LATTICE SPACE BASED ON THE NUMBER OF AMINO ACID RESIDUES IN LINEAR MOLECULAR UNIT

S103 — DEFINE SET OF LATTICE POINTS OF DESTINATION OF i-TH AMINO ACID RESIDUE AS $V_i$

S104 — ALLOCATE SPATIAL INFORMATION TO $X_1$ TO $X_n$

S105 — CREATE ISING MODEL

S106 — OBTAIN MINIMUM VALUE OF E(x) BY ANNEALING MACHINE

S107 — OUTPUT RESULT

# FIG. 16

FIG. 17A

FIG. 17B

## FIG. 17C

## FIG. 17D

# FIG. 18

FIG. 19A

FIG. 19B

# FIG. 19C

# FIG. 20

# FIG. 21

# FIG. 22

FIG. 23A

FIG. 23B

# FIG. 24

|       | $X_1$ | $X_2$ | $X_3$ |
|-------|-------|-------|-------|
| $X_1$ |       | 2     | 0     |
| $X_2$ | 2     |       | 4     |
| $X_3$ | 0     | 4     |       |

# FIG. 25

POSITION LOCATED
WITHIN BRANCHED CHAIN

POSITION LOCATED IN
THE ENTIRE STRUCTURE

1. STRUCTURE SEARCH OF EACH
BRANCHED CHAIN ALONE
(HOLD BINDING STRUCTURE AS
ENTIRE STRUCTURE)

2. CONSTITUTE CONSTRAINTS AND
INTERACTIONS BETWEEN
CONSTITUENT PARTICLES OF
DIFFERENT BRANCHED CHAINS

# FIG. 26

S201 — DIVIDE LINEAR PROTEIN AT DIVIDING POINT

S202 — DEFINE THREE-DIMENSIONAL LATTICE SPACE BASED ON THE NUMBER OF AMINO ACID RESIDUES IN LINEAR MOLECULAR UNIT

S203 — DEFINE SET OF LATTICE POINTS OF DESTINATION OF i-TH AMINO ACID RESIDUE AS $V_i$

S204 — ALLOCATE SPATIAL INFORMATION TO $X_1$ TO $X_n$

S205 — CREATE ISING MODEL

S206 — OBTAIN MINIMUM VALUE OF E(x) BY ANNEALING MACHINE

S207 — OUTPUT RESULT

FIG. 27

100

# FIG. 28

ENERGY CHANGE VALUE $\{-\Delta Ei\}$

114

TRANSITION CONTROL UNIT

114b1

RANDOM
NUMBER
GENERATION
CIRCUIT

114b2

SELECTOR

TRANSITION
NUMBER N

114b3

NOISE
TABLE

114b4

114b5

TRANSITION
ACCEPTANCE f

TEMPERATURE
VALUE T

# FIG. 29

| SELECT ONE STATE TRANSITION AS CANDIDATE | S0001 |

↓

| DETERMINE ACCEPTANCE OF STATE TRANSITION BY COMPARING ENERGY CHANGE VALUE WITH RESPECT TO STATE TRANSITION AND PRODUCT OF TEMPERATURE VALUE AND RANDOM NUMBER VALUE | S0002 |

↓

| ADOPT STATE TRANSITION WHEN ACCEPTED AND NOT ADOPT WHEN NOT ACCEPTED | S0003 |

# FIG. 30

**RELATED ART**

EMBODIMENT 1

NUMBER OF
LATTICES 4

NUMBER OF
LATTICES 2

THE NUMBER OF LATTICES REQUIRED FOR STRUCTURE
SEARCH MAY BE SUPPRESSED TO REDUCE THE NUMBER
OF BITS USED FOR CALCULATION

EMBODIMENT 2

FURTHER ACCURATE STRUCTURE SEARCH IS
POSSIBLE FOR MOLECULES HAVING BRANCHED
STRUCTURE SUCH AS PROTEIN IN
CONSIDERATION OF SIDE CHAIN

Europäisches
Patentamt

European
Patent Office

Office européen
des brevets

**EUROPEAN SEARCH REPORT**

Application Number

EP 20 15 9660

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | SORIN ISTRAIL ET AL: "Combinatorial Algorithms for Protein Folding in Lattice Models: A Survey of Mathematical Results", COMMUNICATIONS IN INFORMATION AND SYSTEMS, vol. 9, no. 4, 17 June 2009 (2009-06-17), pages 303-346, XP55723531, US ISSN: 1526-7555, DOI: 10.4310/CIS.2009.v9.n4.a2 * throughout, in part. sect. 2.2, 3.2, 4 * | 1-13 | INV. G16B15/20 |
| A | SUN-YUAN HSIEH ET AL: "A New Branch and Bound Method for the Protein Folding Problem Under the 2D-HP Model", IEEE TRANSACTIONS ON NANOBIOSCIENCE, IEEE SERVICE CENTER, PISCATAWAY, NY, US, vol. 10, no. 2, 1 June 2011 (2011-06-01), pages 69-75, XP011411624, ISSN: 1536-1241, DOI: 10.1109/TNB.2011.2145388 * throughout, e.g. abstract, pgs. 70-73 * | 1-13 | |
| X | Ryan Babbush ET AL: "Construction of Energy Functions for Lattice Heteropolymer Models: Efficient Encodings for Constraint Satisfaction Programming and Quantum Annealing" In: "Advances in Chemical Physics: Volume 155", 11 April 2014 (2014-04-11), John Wiley & Sons, Inc., Hoboken, New Jersey, XP055692281, ISBN: 978-1-118-75577-8 pages 201-244, DOI: 10.1002/9781118755815.ch05, * throughout, in part. pg. 207 * | 1-13 | TECHNICAL FIELDS SEARCHED (IPC) G16B |

The present search report has been drawn up for all claims

| Place of search | Date of completion of the search | Examiner |
|---|---|---|
| Munich | 19 August 2020 | Wimmer, Georg |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2010113473 A

**Non-patent literature cited in the description**

- **BABBUSH RYAN et al.** Construction of Energy Functions for Lattice Heteropolymer Models: A Case Study in Constraint Satisfaction Programming and Adiabatic Quantum Optimization. *Advances in Chemical Physics,* vol. 155, 201-244